# EUROPÄISCHE PATENTSCHRIFT

(11) **EP 1 003 478 B1**
(45) Veröffentlichungstag und Bekanntmachung des Hinweises auf die Patenterteilung: **17.08.2005**
(21) Anmeldenummer: 98943814.8
(22) Anmeldetag: 31.07.1998
(51) Int. Cl.: A61K 9/00, A61K 38/28

(54) **WÄSSRIGE AEROSOLZUBEREITUNGEN ENTHALTEND BIOLOGISCH AKTIVE MAKROMOLEKÜLE UND VERFAHREN ZUR ERZEUGUNG ENTSPRECHENDER AEROSOLE**
AQUEOUS AEROSOL PREPARATIONS CONTAINING BIOLOGICALLY ACTIVE MACROMOLECULES AND METHOD FOR PRODUCING THE CORRESPONDING AEROSOLS
PREPARATIONS D'AEROSOLS AQUEUSES CONTENANT DES MACROMOLECULES BIOLOGIQUEMENT ACTIVES ET PROCEDE DE PRODUCTION DES AEROSOLS CORRESPONDANTS

(30) Priorität: 04.08.1997 DE 19733651
(43) Veröffentlichungstag der Anmeldung: 31.05.2000
(60) Teilanmeldung: 05002747.3
(73) Patentinhaber: Boehringer Ingelheim Pharma GmbH & Co.KG, 55218 Ingelheim am Rhein (DE)
(72) Erfinder: LAMCHE, Herbert, A-2534 Alland (AT); MEADE, Christopher, John, Montague, D-88437 Maselheim (DE); ZIERENBERG, Bernd, D-55411 Bingen am Rhein (DE); REIMHOLZ, Ralph, Christian, 65195 Wiesbaden (DE)
(74) Vertreter: Kläs, Heinz-Gerd, Dr.
(86) Internationale Anmeldenummer: PCT/EP1998/004803
(87) Internationale Veröffentlichungsnummer: WO 1999/007340

(56) Entgegenhaltungen:
- WO-A-90/09781
- WO-A-92/06704
- WO-A-94/17822
- WO-A-95/03034
- DE-A- 4 117 078

## Beschreibung

Die Erfindung betrifft ein Verfahren zur Erzeugung von Aerosolen für die inhalative Applikation von Proteinen und anderen biologisch aktiven Makromolekülen sowie wäßrige Zubereitungen zur Erzeugung solcher Aerosole, insbesondere betrifft die Erfindung wäßrige Zubereitungen hochkonzentrierter Lösungen von Insulin für die inhalative Applikation zur Behandlung von Diabetes.

Die Anwendung von Arzneistoffen in Form inhalierfähiger Aerosole ist seit langem bekannt. Solche Aerosole dienen nicht nur zur Behandlung von Atemwegserkrankungen wie Asthma; sie werden auch verwendet, wenn die Lunge oder die Nasenschleimhäute als Resorptionsorgan dienen sollen. Häufig können so hohe Blutspiegel des Wirkstoffes erzeugt werden , um auch Krankheiten in anderen Körperregionen zu behandeln. Inhalierbare Aerosole können auch als Impfstoffe dienen.

Zur Herstellung von Aerosolen werden in der Praxis mehrere Verfahren angewendet. Entweder werden Suspensionen oder Lösungen von Wirkstoffen mit Hilfe von Treibgasen versprüht oder Wirkstoffe in Form mikronisierter Pulver in der Atemluft verwirbelt oder schließlich wäßrige Lösungen mit Hilfe von Verneblern zerstäubt.

Doch bei komplizierter gebauten Molekülen, wie z. B. Interferonen, kann die Verneblung wäßriger Lösungen leicht zu einer störenden Verminderung der Wirkstoffaktivität führen, vermutlich durch Scherkräfte und Erwärmung. Es wird vermutet, daß in diesem Prozeß z.B. die Bildung minderaktiver Proteinaggregate eine Rolle spielt. A.Y. Ip und Kollegen haben, in Ihrem Artikel 'Stability of recombinant consensus interferon to air-jet and ultrasonic nebulisation' J. Pharm. Sci. 84: 1210-1214 [1995], Beispiele für die Entstehung von Interferonaggregaten nach Ultraschall oder Düsen-Verneblung, mit damit einhergehendem Verlust der biologischen Aktivität des Interferones beschrieben. Auch wenn die Zerstörung des Biomoleküls (biologisch aktive Makromoleküle) nicht vollständig ist, ist die hier beschriebene Aktivitätsverminderung wichtig, weil sie einen größeren Verbrauch des möglicherweise teuren Biomoleküles verursacht und die Dosierung an aktivem Arzneimittel pro Hub ungenau werden läßt. Diese Abnahme der Aktivität von kompliziert gebauten Molekülen während der Aerosolerzeugung ist nicht nur auf Interferone begrenzt, sie kommt in kleinerem oder größerem Maß auch während der Aerosolisierung anderer Proteine (siehe z.B. Niven et al, Pharm Res. 12: 53-59 [1995]) und Biomoleküle vor.

Neben der technischen Herstellung des das Biornolekül enthaltenden Aerosols ist ein zweiter Schritt notwendig, damit die Biomoleküle in der Lunge absorbiert werden. Die Lunge des erwachsenen Menschen bietet eine große Absorptionsfläche, aber sie weist auch mehrere Hindernisse für die pulmonäre Absorption von Biomolekülen auf. Nach Inspiration durch die Nase oder den Mund geht Luft mit dem von ihr getragenen Aerosol in die Trachea und dann über immer kleinere Bronchien und Bronchiolen in die Alveoli. Die Alveoli haben eine viel größere Fläche als Trachea, Bronchi und Bronchiolen zusammen. Sie sind die Hauptabsorptionszone, nicht nur für Sauerstoff, sondern auch für biologisch aktive Makromoleküle. Um aus der Luft in die Blutbahn zu gelangen, müssen Moleküle das alveoläre Epithel, das kapilläre Endothel und den lymphe-enthaltenden interstitiellen Raum zwischen diesen zwei Zell-Schichten durchqueren. Dies kann über aktive oder passive Transportprozesse stattfinden. Die Zellen in diesen zwei Zell-Schichten liegen dicht beieinander, so daß die meisten großen biologischen Makromoleküle (wie z.B. Proteine) diese Sperre viel langsamer durchqueren als kleinere Moleküle. Der Prozeß der Durchquerung des alveolären Epithels und des kapillären Endothels läuft in Konkurrenz mit anderen biologischen Prozessen die zur Zerstörung des Biomoleküls führen. Die bronchoalveoläre Flüssigkeit enthält Exoproteasen [siehe z.B. Wall D.A. und Lanutti, A.T. 'High levels of exopeptidase activity are present in rat and canine bronchoalveolar lavage fluid'. Int.J.Pharm. 97:171-181 (1993)]. Sie enthält auch Macrophagen, welche inhalierte Protein-Teilchen mittels Phagozytose eliminieren. Diese Macrophagen migrieren zur Basis des bronchialen Baumes, von wo aus sie mittels des mucoziliaren Clearance Mechanismus aus der Lunge geraten. Sie können dann in die Lymphbahn migrieren. Weiter können die Macrophagen vom aerosolisierten Protein in ihrer Physiologie beeinflußt werden, z.B. können Interferone alveolare Macrophagen aktivieren. Die Migration von aktivierten Macrophagen stellt einen weiteren Mechanismus für die Verbreitung der systemischen Wirkung eines inhalierten Proteins dar. Die Komplexität dieses Prozesses bedeutet, daß Ergebnisse von Aerosol-Versuchen mit einem Protein Typ auf einen anderen Protein Typ nur begrenzt übertragbar sind. Kleine Unterschiede zwischen Interferonen können zum Beispiel einen deutlichen Einfluß auf Ihre Susceptibilität gegenüber den Degradationsmechanismen in der Lunge haben [siehe Bocci et al 'Pulmonary catabolism of interferons: alveolar absorption of ¹²⁵-I labelled human interferon alpha is accompanied by partial loss of biological activity' Antiviral Research 4:211-220 (1984)].

Proteine und andere biologische Makromoleküle können zwar prinzipiell vernebelt werden, aber diese Verneblung findet in der Regel mit einem Aktivitätsverlust statt. Es ist die Aufgabe der vorliegenden Erfindung ein Verfahren für die Herstellung von inhalierbaren Aerosolen zur Verfügung zu stellen, mit dem biologisch aktive Makromoleküle, insbesondere Proteine, ohne wesentliche Aktivitätsverluste vernebelt werden können.

Eine neue Generation von treibgasfreien Verneblern ist in dem US-Patent 5,497,944 beschrieben, auf das hiermit inhaltlich Bezug genommen wird. Der besondere Vorteil der dort beschriebenen Vernebler ist, daß auf die Verwendung von Treibgasen, insbesondere von Fluorochlorkohlenwasserstoffen, verzichtet wird.

Eine weiterentwickelte Ausführungsform der dort beschriebenen Vernebler ist in der PCT/EP96/04351 = WO 97/12687 offenbart. In Bezug auf die vorliegende Erfindung wird ausdrücklich auf die dort beschriebene Figur 6 (Respimat®) sowie auf die dazugehörigen Beschreibungsteile der Anmeldung verwiesen. Der dort beschriebene Vernebler kann vorteilhaft zur Erzeugung der erfindungsgemäßen inhalierbaren Aerosole biologisch aktiver Makromoleküle eingesetzt werden. Insbesondere kann der beschriebene Vernebler zur inhalativen Applikation von Insulin eingesetzt werden. Aufgrund seiner handlichen Größe kann dieses Device jederzeit vom Patienten mitgeführt werden. Bei dem dort beschriebenen Vernebler werden wirkstoffhaltige Lösungen definierter Volumina (bevorzugt ca. 15 Mikroliter) unter Anwendung hoher Drucke durch kleine Düsen versprüht, so daß inhalierbare Aerosole mit einer mittleren Teilchengröße zwischen 3 und 10 Mikrometer entstehen. Für die Inhalative Applikation von Insulin sind Vernebler geeignet, die zwischen 10 und 50 Mikroliter Aerosolzubereitung pro Anwendung zu inhalierbaren Tröpfchen vernebeln können.

Von besonderer Bedeutung zur Herstellung der erfindungsgemäßen Aerosole ist die Verwendung des in dem oben beschriebenen Patent bzw. Patentanmeldung beschriebenen Verneblers zum treibgasfreien Zerstäuben von wirkstoffhaltigen Lösungen, die Proteine oder andere biologisch aktive Makromoleküle enthalten.

Im wesentlichen besteht der dort offenbarte handhabbare Zerstäuber (Vernebler, Größe ca. 10 cm) aus einem Gehäuseoberteil, einem Pumpengehäuse, einer Düse, einem Sperrspannwerk, einem Federgehäuse, einer Feder und einem Vorratsbehälter, gekennzeichnet durch
- ein Pumpengehäuse, das im Gehäuseoberteil befestigt ist, und das an seinem einen Ende einen Düsenkörper mit der Düse bzw. Düsenanordnung trägt,
- einen Hohlkolben mit Ventilkörper,
- einen Abtriebsflansch, in dem der Hohlkolben befestigt ist, und der sich im Gehäuseoberteil befindet,
- ein Sperrspannwerk, das sich im Gehäuseoberteil befindet,
- ein Federgehäuse mit der darin befindlichen Feder, das am Gehäuseoberteil mittels eines Drehlagers drehbar gelagert ist,
- ein Gehäuseunterteil, das auf das Federgehäuse in axialer Richtung aufgesteckt ist.

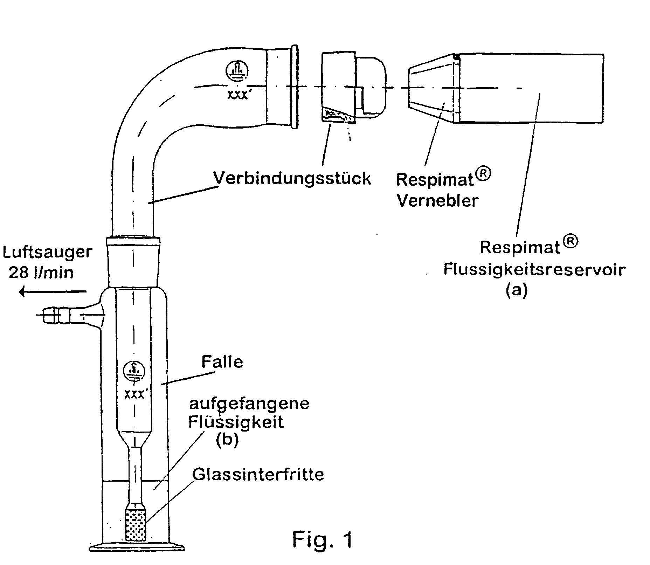

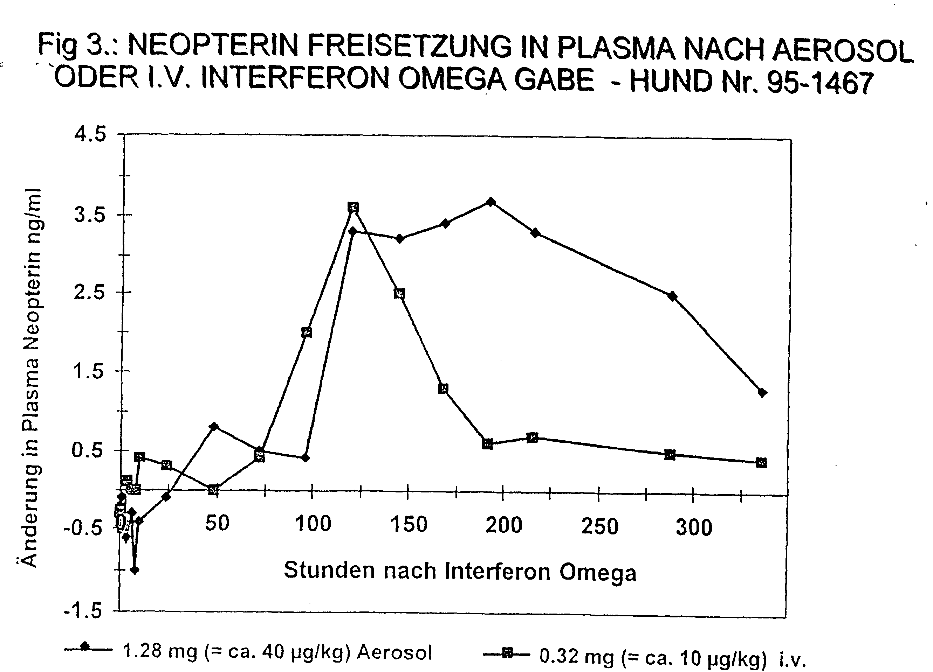

Der Hohlkolben mit Ventilkörper WO 97/12687 entspricht einer dort der offenbarten Vorrichtungen. Er ragt teilweise in den Zylinder des Pumpengehäuses hinein und ist im Zylinder axial verschiebbar angeordnet. Insbesondere wird auf die Figuren 1-4 - insbesondere Figur 3 - und die dazugehörigen Beschreibungsteile Bezug genommen. Der Hohlkolben mit Ventilkörper übt auf seiner Hochdruckseite zum Zeitpunkt des Auslösens der Feder einen Druck von 5 bis 60 Mpa (etwa 50 bis 600 bar), bevorzugt 10 bis 60 Mpa (etwa 100 bis 600 bar) auf das Fluid, die abgemessene Wirkstofflösung aus.

Der Ventilkörper ist bevorzugt an dem Ende des Hohlkolbens angebracht, das dem Düsenkörper zugewandt ist.

Die Düse im Düsenkörper ist bevorzugt mikrostrukturiert, d.h. durch Mikrotechnik hergestellt. Mikrostrukturierte Düsenkörper sind beispielsweise in WO-94/07607 offenbart; auf diese Schrift wird hiermit inhaltlich Bezug genommen.

Der Düsenkörper besteht z. B. aus zwei fest miteinander verbundenen Platten aus Glas und/oder Silizium, von denen wenigstens eine Platte einen oder mehrere mikrostrukturierte Kanäle aufweist, die die Düseneinlaßseite mit der Düsenauslaßseite verbinden. Auf der Düsenauslaßseite ist mindestens eine runde oder nicht-runde Öffnung von kleiner oder gleich 10 µm angebracht.

Die Strahlrichtungen der Düsen im Düsenkörper können parallel zueinander verlaufen oder gegeneinander geneigt sein. Bei einem Düsenkörper mit mindestens zwei Düsenöffnungen auf der Auslaßseite können die Strahlrichtungen wird ein Winkel von 20 Grad bis 160 Grad gegeneinander geneigt sein, bevorzugt wird ein Winkel von 60 bis 150 Grad. Die Strahlrichtungen treffen sich in der Umgebung der Düsenöffnungen.

Das Sperrspannwerk enthält eine Feder, bevorzugt eine zylindrische schraubenförmige Druckfeder, als Speicher für die mechanische Energie. Die Feder wirkt auf den Abriebsflansch als Sprungstück, dessen Bewegung durch die Position eines Sperrglieds bestimmt wird. Der Weg des Abriebsflansches wird durch einen oberen und einen unteren Anschlag präzise begrenzt. Die Feder wird bevorzugt über ein kraftübersetzendes Getriebe, z.B. ein Schraubschubgetriebe, durch ein äußeres Drehmoment gespannt, das beim Drehen des Gehäuseoberteils gegen das Federgehäuse im Gehäuseunterteil erzeugt wird. In diesem Fall enthalten das Gehäuseoberteil und der Abtriebsflansch ein ein- oder mehrgängiges Keilgetriebe.

Das Sperrglied mit einrückenden Sperrflächen ist ringförmig um den Abtriebsflansch angeordnet. Es besteht z.B. aus einem in sich radial elastisch verformbaren Ring aus Kunststoff oder aus Metall. Der Ring ist in einer Ebene senkrecht zur Zerstäuberachse angeordnet. Nach dem Spannen der Feder schieben sich die Sperrflächen des Sperrgliedes in den Weg des Abtriebsflansches und verhindern das Entspannen der Feder. Das Sprerrglied wird mittels einer Taste ausgelöst. Die Auslösetaste ist mit dem Sperrglied verbunden oder gekoppelt. Zum Auslösen des Sperrspannwerkes wird die Auslösetaste parallel zur Ringebene, und zwar bevorzugt in den Zerstäuber hinein, verschoben; dabei wird der verformbare Ring in der Ringebene verformt. Konstruktive Details des Sperrspannwertes sind in der WO 97/20590 beschrieben.

Das Gehäuseunterteil wird in axialer Richtung über das Federgehäuse geschoben und verdeckt die Lagerung, den Antrieb der Spindel und den Vorratsbehälter für das Fluid.

Beim Betätigen des Zerstäubers wird das Gehäuseobereil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganzzahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgezogen, wodurch eine Teilmenge des Fluids aus dem Vorratsbehälter in den Hochdruckraum vor der Düse eingesaugt wird.

In den Zerstäuber können gegebenenfalls nacheinander mehrere das zu zerstäubende Fluid enthaltende austauschbare Vorratsbehälter eingeschoben und benutzt werden. Der Vorratsbehälter enthält die erfindungsgemäße wässerige Aerosolzubereitung.

Der Zerstäubungsvorgang wird durch leichtes Eindrücken der Auslösetaste eingeleitet. Dabei gibt das Sperrwerk den Weg für das Abtriebsteil frei. Die gespannte Feder schiebt den Kolben in den Zylinder des Pumpengehäuses hinein. Das Fluid tritt aus der Düse des Zerstäubers in zerstäubter Form aus.

Weitere konstruktive Details sind in den PCT-Anmeldungen WO 97/12683 und WO 97/20590 offenbart, auf die hiermit inhaltlich Bezug genommen wird.

Die Bauteile des Zerstäubers (Verneblers) sind aus einem der Funktion entsprechend geeignetem Material. Das Gehäuse des Zerstäubers und - so weit es die Funktion erlaubt - auch andere Teile sind bevorzugt aus Kunststoff, z.B. im Spritzgießverfahren, hergestellt. Für medizinische Zwecke werden physiologisch unbedenkliche Materialien verwendet.

Der in der WO 97/12687 beschriebene Zerstäuber wird z.B. zum treibgasfreien Erzeugen medizinischer Aerosole benutzt. Damit läßt sich ein inhalierbares Aerosol mit einer mittleren Tröpfchengröße von etwa 5 µm erzeugen.

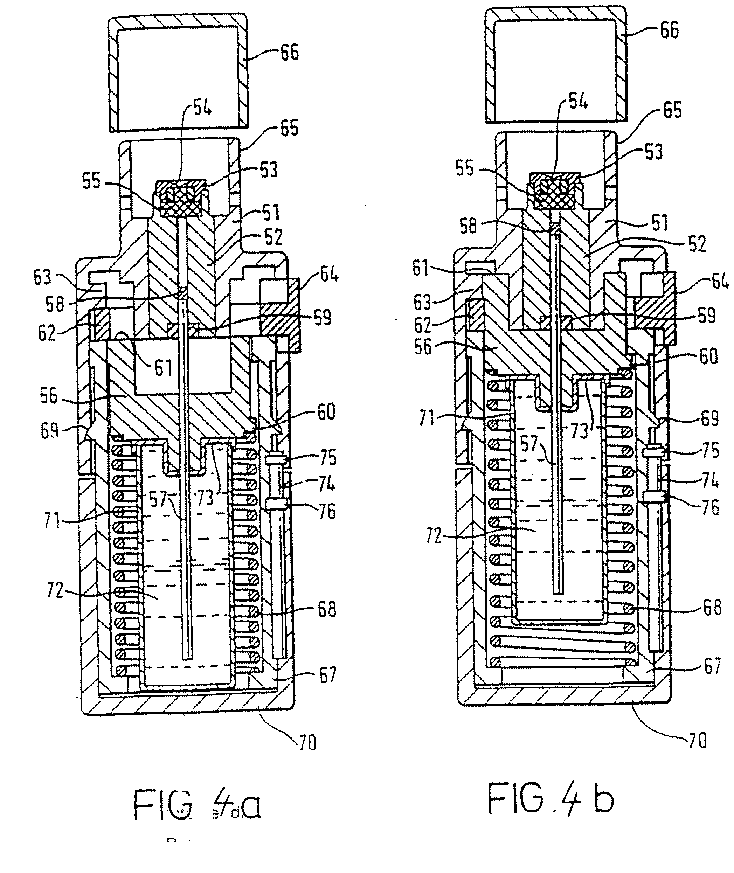

In den Figuren 4 a/b die identisch mit den Figuren 6 a/b der WO 97/12687 sind, ist der Vernebler (Respimat®) beschrieben, mit dem die erfindungsgemäßen wäßrigen Aerosolzubereitungen vorteilhaft inhaliert werden können.

Figur 4 a zeigt einen Längsschnitt durch den Zerstäuber bei gespannter Feder, Figur 4 b zeigt einen Längsschnitt durch den Zerstäuber bei entspannter Feder.

Das Gehäuseoberteil (51) enthält das Pumpengehäuse (52), an dessen Ende der Halter (53) für die Zerstäuberdüse angebracht ist. In dem Halter befindet sich der Düsenkörper (54) und ein Filter (55). Der im Abtriebsflansch (56) des Sperrspannwerkes befestigte Hohlkolben (57) ragt teilweise in den Zylinder des Pumpengehäuses hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespanner Feder anliegt. Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseobereil endet im Mundstück (65) und ist mit der aufsteckbaren Schutzkappe (66) verschlossen.

Das Federgehäuse (67) mit Druckfeder (68) ist mittels der Schnappnasen (69) und Drehlager am Gehäuseoberteil drehbar gelagert. Über das Federgehäuse ist das Gehäueunterteil (70) geschoben. Innerhalb des Federgehäuses befindet sich der austauschbare Vorratsbehälter (71) für das zu zerstäubende Fluid (72). Der Vorratsbehälter ist mit dem Stopfen (73) verschlossen, durch den der Hohlkolben in den Vorratsbehälter hineinragt und mit seinem Ende in das Fluid (Vorrat an Wirkstofflösung) eintaucht.

In der Mantelfläche des Federgehäuses ist die Spindel (74) für das mechanische Zählwerk angebracht. An dem Ende der Spindel, das dem Gehäuseoberteil zugewandt ist, befindet das Antriebsritzel (75). Auf der Spindel sitzt der Reiter (76).

Der oben beschriebene Vernebler ist geeignet, die erfindungsgemäßen Aerosolzubereitungen zu einem für die Inhalation geeignetem Aerosol zu vernebeln.

Die Effektivität eines Verneblungsgerätes kann in einem *in vitro* System getestet werden, in dem eine Proteinlösung vernebelt wird und der Nebel in einer sogenannten 'Falle' (siehe Fig. 1) abgefangen wird. Die Aktivität des Proteins im Aerosolreservoir (a) wird mit der Aktivität in der aufgefangenen Flüssigkeit (b) verglichen, z.B: mit Hilfe eines Immunoassays oder mit Hilfe eines Assays für die biologische Wirksamkeit des Proteins. Dieser Versuch erlaubt eine Beurteilung des Grades der Zerstörung des Proteins durch Verneblung. Ein zweiter Parameter der Aerosolqualität ist der sogenannte inhalierbare Anteil, der hier als Anteil der Nebeltröpfchen mit einem Maßmedianen aerodynamischen Durchmesser (MMAD) von weniger als 5.8 µm definiert ist. Der inhalierbare Anteil kann mittels eines "Andersen Impactors" gemessen werden. Für eine gute Proteinabsorption ist es wichtig, nicht nur eine Verneblung ohne wesentlichen Aktivitätsverlust zu erreichen, sondern ein Aerosol mit einem guten (ca. 60%) inhalierbaren Anteil zu generieren. Aerosole mit einem MMAD von weniger als 5.8 µm sind deutlich besser geeignet die Alveoli zu erreichen, wo ihre Chancen absorbiert zu werden deutlich größer sind. Die Effektivität eines Verneblungsgerätes kann auch in einem in vivo System getestet werden, wobei in diesem Fall Faktoren wie Susceptibilität gegenüber Lungenproteasen im Spiel sind. Als Beispiel eines in vivo Testsystems kann einem Hund ein Protein-enthaltender Nebel über einen Tracheal Tubus verabreicht werden. Blutproben werden in passenden Zeitabständen genommen und danach wird der Proteinspiegel im Plasma mit immunologischen oder biologischen Methoden gemessen.

Geeignete Vernebler sind in der bereits oben genannten US-Patentschrift 5,497,944 und der WO97/12687 insbesondere wie in den Figuren 6 a/b (jetzt 4 a/b) beschrieben. Eine bevorzugte Düsenanordnung für die Verneblung der erfindungsgemäßen wäßrigen Aerosolzubereitungen biologisch aktiver Makromoleküle ist in Figur 8 der US-Patentschrift dargestellt.

Überraschenderweise wurde gefunden, daß der oben beschriebene treibgasfreie Vernebler, der eine vorherbestimmte Menge - beispielsweise 15 Mikroliter - einer Aerosolzubereitung unter hohem Druck zwischen 100 und 500 bar durch mindestens eine Düse mit einem hydraulischen Durchmesser von 1 bis 12 Mikrometer versprüht, so daß inhalierbare Tröpfchen mit einer mittleren Teilchengröße kleiner als 10 Mikrometer entstehen, für die Verneblung flüssiger Aerosolzubereitungen von Proteinen und anderen Makromolekülen gut geeignet ist, weil er eine breite Palette von Proteinen ohne nennenswerte Aktivitätsverluste vernebeln kann. Bevorzug ist dabei eine Düsenanordnung wie sie in der Figur 8 der oben genannten US-Patentschrift angegeben ist.

Besonders überraschend ist die Fähigkeit von Verneblern dieser Bauart, Interferone zu vernebeln, welche sonst nur mit beträchtlichem Aktivitätsverlust vernebelt werden können. Weiter überraschend ist die besonders hohe Aktivität von Interferon Omega nach Verneblung mit diesem Gerät, nicht nur in *in vitro* sondern auch in *in vivo* Versuchen.

WO 95/03034 befaßt sich ebenfalls mit der Stabilisierung wäßriger Proteinaerosole. Löst das Problem jedoch durch organische polare Verbindungen oder oberflächenaktive Substanzen.

Eine weiterer Vorteil des beanspruchten Verfahrens ist seine überraschende Eigenschaft, auch hochkonzentrierte Lösungen von biologisch aktiven Makromolekülen ohne wesentlichen Aktivitätsverlust vernebeln zu können.

Der Gebrauch hochkonzentrierter Lösungen ermöglicht die Verwendung eines Gerätes, das klein genug ist, um bequem in einer Jackentasche oder Handtasche ständig getragen zu werden. Der in Figur 4 offenbarte Vernebler erfüllt diese Voraussetzungen und ermöglicht die Verneblung hochkonzentrierter Lösungen biologisch aktiver Moleküle.

Die vorliegende Erfindung ist durch die Ansprüche definiert und bezieht sich auf einen Treibgasfreier Vernebler enthaltend eine Aerosolzubereitung, dessen Verwendung zur Herstellung eines Aerosols sowie auf ein Verfahren zur Herstellung Treibgasfreier Aerosole.

Beispielsweise sind solche Geräte besonders geeignet, um Diabetikern die inhalative Selbstbehandlung mit Insulin zu ermöglichen. Bevorzugt werden hochkonzentrierte wässerige Lösungen mit einer Konzentration von 25 bis 90 mg/ml Insulin eingesetzt, bevorzugt sind Lösungen mit 30 bis 60 mg/ml Insulin und besonders bevorzugt sind Insulinlösungen mit 33 bis 40 mg/ml Insulin. Je nach Größe des zur Verfügung steheden Reservoirs des Verneblers sind Lösungen, die Insulin in einer Konzentration von mehr als 25 mg/ml , bevorzugt mehr als 30mg/ml enthalten, geeignet, um eine therapeutisch wirksame Menge Insulin mit einem Handgerät, wie es das oben beschriebene Device darstellt, inhalieren zu können. Die inhalative Applikation von Insulin ermöglicht einen schnellen Wirkungseintritt der Wirksubstanz, so daß der Patient beispielsweise kurz vor den Mahlzeiten die für ihn erforderliche Menge sich selbst applizieren kann. Die geringe Größe des Respimats® beispielsweise ermöglicht es dem Patienten das Gerät jederzeit mit sich zu führen.

Der Respimat® (Figur 6 der WO 97/12687) verfügt über eine Dosierkammer mit konstantem Volumen, die es dem Patienten ermöglicht, die für ihn erforderliche Dosis an Insulin durch die Anzahl der Hübe (puffs) zu bestimmen und zu inhalieren. Neben der Anzahl der Hübe ist die Dosierung des Insulins durch die Konzentration der Insulinlösung in dem Vorratsgefäß (72) bestimmt. Sie kann beispielsweise zwischen 25 und 90 mg/ml betragen, bevorzugt sind höher konzentrierte Lösungen ab ca. 30 mg/ml und mehr.

Verfahren zur Herstellung hochkonzentrierter stabiler Insulinlösungen sind beipielsweise in den WO-Anmeldungen 83/00288 (PCT/DK82/00068) und 83/03054 (PCT/DK 83/00024) beschrieben auf die hiermit inhaltlich Bezug genommen wird.

Erfindungsgemäße Aerosolzubereitungen die Insulin enthalten, die mit dem oben beschriebenen Device appliziert werden, sollten eine dynamische Vikosität von über 1600·10⁻⁶ Pa s nicht überschreiten, damit der inhalierbare Anteil des erzeugten Sprays nicht unter einen akzeptablen Wert sinkt. Bevorzugt sind Insulinlösungen, die eine Grenzvikosität bis zu 1200·10⁻⁶, besonders bevorzugt bis zu 1100·10⁻⁶ Pa s (Pascal Sekunde) aufweisen. Falls erforderlich sind anstelle von Wasser als Lösemittel Lösungsmittelgemische verwendbar, um die Viskosität der Arzneimittellösung herabzusetzen. Dies kann beispielsweise durch den Zusatz von Ethanol erfolgen. Der Anteil an Ethanol an der wäßrigen Formulierung kann beispielsweise bis zu 50% betragen, bevorzug ist ein Anteil von 30 %.

Vorzugsweise weist die Aerosolzubereitung eine Viskosität von bis zu 1600·10⁻⁶ Pa·s auf, wobei ein Bereich von 900 bis 1100·10⁻⁶ Pa·s besonders bevorzugt ist.

Bevorzugt sind ferner Aerosolzubereitungen, deren wäßrige Lösungen eine Viskosität zwischen 900 und 1600·10⁻⁶ Pa·s aufweisen, worunter wässrige Lösungen mit einer Viskosität in einem Bereich von 950 und 1300·10⁻⁶ Pa·s besonders bevorzugt sind.

Es ist eine weitere Aufgabe der vorliegenden Erfindung eine geeignete Aerosolzubereitung vorzuschlagen, die zur Verwendung in den beanspruchten Verfahren geeignet ist.

Zum Gegenstand der Erfindung gehören ferner Aerosolzubereitungen in Form einer Lösung mit Wasser oder einem Wasser-Ethanol-Gemisch als Lösungsmittel, die als Wirkstoff biologisch aktive Makromoleküle, insbesondere ein Protein oder ein Peptid, in einer Konzentration von 25 bis 100 mg/ml enthalten, wobei der Wirkstoff ausgewählt ist aus der Gruppe Antisense Oligonukleotide, Orexine, Eryhtropoetin, Tumor Nekrose Faktor alpha; Tumor Nekrose Faktor-beta; GM-CSF ("Granulocyte-macrophage colony stimulating factor") Annexine; Calcitonin; Leptine; Parathormone; Parathormone Fragement; Interleukine wie z.B., Interleukin 2, Interleukin 10, Interleukin 12; Insulin Superoxid Dismutase; ein Interferon; lösliches ICAM ("Interceullar adhesion molecule"); Somatostatin; Somatotropin; tPA("Tissue plasminogen activator") TNK-tPA; Tumorassozierte Antigene (als Peptid, Protein oder als DNA) Peptid Bradykinin Antagonisten; Urodilatin; GHRH (Growth hormone releasing hormone); CRF (Corticotropin releasing factor); EMAP II Heparin; lösliche Interteukin Rezeptoren wie sIL-1 Rezeptor; Impfstoffe wie Hepatitis Vakzine oder Masern Vakzine; Antisense Polynukleotide Transkriptions Faktoren, zur Herstellung eines inhalierbaren Aerosols mittels eines treibgasfreien Vemeblers.

Überraschenderweise hat sich gezeigt, daß nach dem erfindungsgemäß beanspruchten Verfahren auch höher viskose Lösungen von Makromolekülen zu inhalierbaren Tröpfchen geeigneter Tröpfchengröße versprüht werden können. Dies ermöglicht die Applikation größerer Wirkstoffmengen pro Anwendung und erhöht damit die therapeutische Wirksamkeit von Makromolekülen bei der inhalativen Therapie.

Nach dem erfindungsgemäßen Verfahren können Makromoleküle (zum Beispiel Albumin) enthaltende wäßrige Aerosolzubereitungen bis zu einer Viskosität von 1600·10⁻⁶ Pa s (gemessen bei 25°C) eingesetzt werden. Bei einer Viskosität von 1500·10⁻⁶ Pa s wurde noch ein inhalierbarer Anteil von 32 % ermittelt.

Bevorzugt sind höhere Viskose Lösungen von Makromotekülen, die eine Viskosität bis zu 1100·10⁻⁶ Pa s aufweisen. Bei solchen Lösungen wird ein inhalierbarer Anteil an wirkstoffhaltigen Tröpfchen von ca. 60 % errreicht. Die angegebenen Grenzviskositäten wurden mit einem Ostwald Viskosimeter nach literaturbekannter Methode bestimmt. Zum Vergleich, die Viskosität von Wasser liegt bei 894·10⁻⁶ Pa s (gemessen bei 25°C).

Um Vorteile des erfindungsgemäßen Verfahrens zu illustrieren, werden im folgenden *in vitro* und *in vivo* Versuche mit einer Interferon Omega Lösung beschrieben.

### In vitro Versuche mit Respimat® und Interferon Omega

Das Reservoir eines Respimat Gerätes (a) wurde mit einer 5mg/ml Interferon Omega Lösung (in 50 mM Trinatriumcitrat, 150 mM NaCl, pH 5.5 formuliert, gefüllt (wobei die angegebene Wirkstoffkonzentration vom Wortlaut nach Anspruch 1 abweicht). Das Gerät wurde aktiviert, und ein Volumen von ca. 12.9 µl (ein Hub) in einem Luftstrom von 28 l/Min vernebelt. Die vernebelte Lösung wurde in einer Falle (Fig. 1) gefangen. Interferon Omega wurde in der Reservoirlösung und in der gefangenen Falle Lösung, immunologisch, mittels eines ELISAs, und biologisch, durch die Hemmung der Zerstörung von Encephalo-Myocarditis Virus infizierten A549 Zellen, bestimmt. Die immunologische Bestimmung von Interferon ist relativ einfach. Veröffentlichte Untersuchungen mit vernebelten Proteinen sind in mehreren Fällen auf immunologische Messungen beschränkt. Zusätzliche biologische Messungen sind jedoch sehr wichtig, weil sie eine besonders empfindliche und therapeutisch relevante Methode der Quantifizierung der Proteinzerstörung sind. Sie geben nicht immer das gleiche Ergebnis wie physiko-chemische oder immunologische Methoden, weil ein Molekül biologische Fähigkeiten verlieren kann, ohne daß sich seine Bindung an Antikörper verändert.

In drei Versuchen wurde, bezogen auf die Ausgangslösung, 84%, 77% und 98% des immunologisch identifizierbaren Interferons in der Falle Lösung (b) wiedergefunden. Biologische Messungen mit den gleichen Lösungen ergaben 54%, 47% und 81 % Wiederfindung des biologisch identifizierbaren Interferons in der Falle Lösung. Dieser sehr hohe Anteil zeigt, daß die Verneblung mittels des Respimat Gerätes nur einen relativ kleinen Teil der Aktivität des Interferons zerstört. Der Nebel von einem Respimat Gerät, wie oben beschrieben, wurde auch in einen Andersen Impactor mittels eines Luftstroms (28 l/Min) geleitet. Der Anteil von Teilchen mit einer Größe von weniger als 5.8 µm ('inhalierbarer Anteil') wurde gemessen. Der inhalierbare Anteil entsprach 70% (immunologische Messungen). Proteine wie Interferone sind oft mit Human Serum Albumin formuliert, um einen weiteren Schutz für die empfindlichen Interferone zu bieten. Eine Formulierung wie oben, aber mit zusätzlichem Human Serum Albumin (0.5%) wurde auch getestet. In drei Versuchen wurde ebenso bezogen auf die Ausgangslösung, 83%, 83% und 79% des immunologisch identifizierbaren Interferons in der Falle Lösung (b) wiedergewonnen. Biologische Messungen mit den gleichen Lösungen ergaben 60%, 54% und 66% des biologisch aktiven Interferons in der Falle Lösung. Der inhalierbare Anteil (immunologische Messungen) war 67%. In einem weiteren Ansatz wurde eine konzentrierte Interferon Omega Lösung mit einer Konzentration von 53 mg/ml in das Reservoir des Respimat Gerätes gefüllt und anschließend vernebelt. In vier Versuchen wurde, bezogen auf die Ausgangslösung, 100%, 60%, 68% und 72% des immunologisch identifizierbaren Interferons in der Falle Lösung (b) wiedergefunden. Biologische Messungen mit den gleichen Lösungen ergaben 95%, 98%, 61 % und 83% Wiederfindung des biologisch identifizierbaren Interferons in der Falle Lösung. Diese hohe Wiederfindung zeigt, daß mittels des Respimat Gerätes auch konzentrierte Proteinlösungen vernebelt werden können, ohne daß es dabei zu übermäßigen Verlusten der Interferonaktivität kommt.

### In vivo Versuche mit Respimat® und Interferon Omega

Interferon Omega wurde inhalativ und intravenös in getrennten Versuchen am gleichen Hund appliziert. Es wurden die Blutspiegel des Interferons zu verschiedenen Zeitpunkten immunologisch und biologisch gemessen. Weiters wurde der Neopterinspiegel im Blut gemessen. Neopterin ist ein Marker für Immunaktivierung; es wird von Macrophagen nach Interferon-Reiz freigesetzt [siehe Fuchs et al 'Neopterin, biochemistry and clinical use as a marker for cellular immune reactions' Int. Arch. Allergy Appl. Immunol. 101: 1-6 (1993)]. Die Messung des Neopterinspiegels dient zur Quantifizierung der Interferonwirksamkeit.

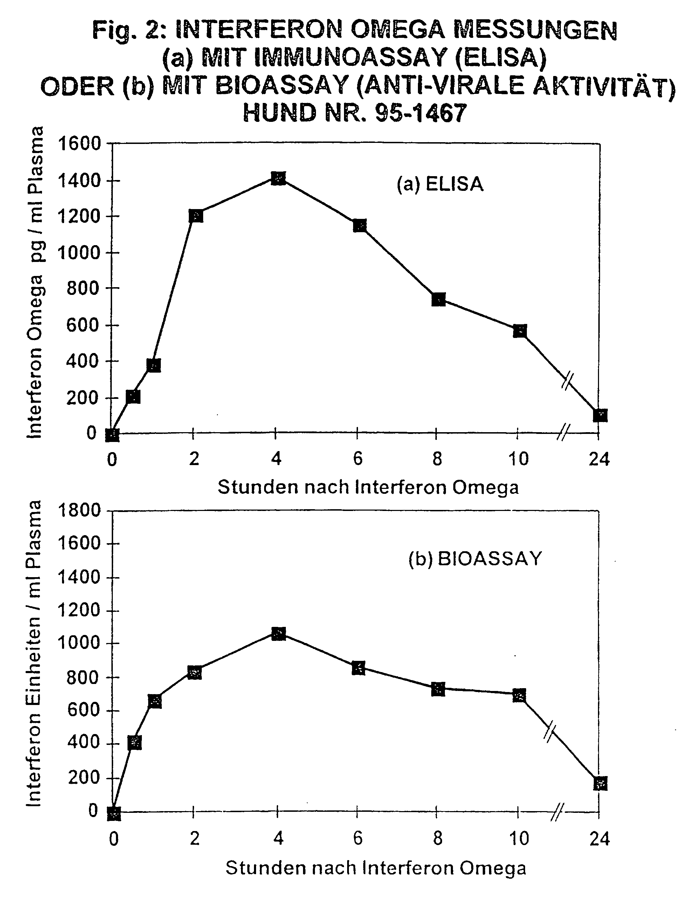

Die Applikation des Interferons beim Hund erfolgte unter Pentobarbitalnarkose nach vorheriger Basissedation. Das Tier wurde intubiert und künstlich beatmet (Volumen-kontrollierte Beatmung: Minutenvolumen 4l/Min, Frequenz 10 Hübe/Min.). Insgesamt 20 Hübe wurden vom Respimat Gerät geliefert. Jeder Hub wurde zu Beginn einer Inspiration appliziert. Nach der Inspirationsphase gab es 5 Sekunden Pause vor der Expiration. Vor der nächsten Interferon Omega Applikation wurden dem Tier zwei Atemzyklen ohne Beeinflussung erlaubt. Blut für Serum und Heparin-Plasma wurde vor Interferon Applikation und zu verschiedenen Zeitpunkten bis 14 Tage nach der Interferon Applikation entnommen. Interferon Omega wurde in Heparin-Plasma immunologisch mittels eines ELISAs, und biologisch durch die Hemmung der Zerstörung von Encephalo-Myocarditis Virus infizierten A549 Zellen, bestimmt. Serum-Neopterin wurde immunologisch bestimmt. Figur 2 zeigt die immunologisch (Fig. 2a) und biologisch (Fig. 2b) gemessenen Interferon Omega Spiegel nach 20 Hüben Interferon Omega vom Respimat Gerät. Überraschenderweise wurde nach inhalativer Gabe ein sehr hoher Serum Neopterin Spiegel gemessen. Im *in vitro* Versuch entsprach das ausgebrachte Maß von Lösung nach einem Hub des Respimat Gerätes durchschnittlich 12,8 mg/Hub. Deshalb ist zu erwarten, daß bei einer 5 mg/ml Lösung mit 20 Hüben des Respimates ca. 1,28 mg Interferon geliefert werden. Neopterin Messungen nach Gabe dieser Menge ergaben deutlich höhere und länger andauernde Spiegel, als Neopterin Messungen nach der Gabe von 0,32 mg Interferon i.v. Fig. 3 illustriert dieses Ergebnis. Die hohen Neopterin Spiegel dienen als Beweis, daß die Applikation von Interferon durch den Respimat zu einer guten biologischen Wirksamkeit führen kann.

Die Vorteile des Respimat Gerätes für die Verneblung von biologischen aktiven Makromolekülen ist nicht nur auf Interferone begrenzt, wie ein zweites Beispiel zeigt.

### In vitro Versuche mit Respimat® und Mangan Superoxid Dismutase

Das Gerät für die Veneblung der Test Substanz und die dazugehörige Falle war wie in Fig. 1 dargestellt. In diesem Versuch wurde das Reservoir (a) des Respimat Gerätes mit 3.3 mg/ml Mangan Superoxid Dismutase (MnSOD) in phosphat gepufferter Salzlösung (PBS), gefüllt (wobei die angegebene Wirkstoffkonzentration vom Wortlaut nach Anspruch 1 abweicht). Das Gerät wurde aktiviert, und ein Volumen von ca. 13 µl (ein Hub) in einem Luftstrom von 28 l/Min vernebelt. Die genaue vernebelte Menge wurde gravimetrisch bestimmt (Messungen in drei aufeinander folgenden Versuchen: 12,8, 13,7 und 14,3 mg). Die vernebelte Lösung wurde in einer Falle (b) gefangen. Inhalt dieser Falle war 20 ml PBS. Zusätzlich wurden 2 ml 5% Rinder Serum Albumin zugegeben, um Proteine in der Falle zu stabilisieren. MnSOD wurde in der Reservoirlösung und in der gefangenen Falle Lösung, immunologisch mittels eines ELISAs, und enzymatisch durch die Reduktion der Superoxidmenge nach einer Xanthin/Xanthin-Oxidase Reaktion bestimmt. In drei Versuchen wurde 78%, 89% und 83% der immunologisch identifizierbaren MnSOD der vernebelten Lösung in der Falle Lösung (b) gemessen. Es gab keinen messbaren Verlust der enzymatischen Aktivität nach Verneblung. Der inhalierbare Anteil (immunologische Messungen) war 61%.

Im nachfolgenden Beispiel wird die Herstellung einer erfindungsgemäßen Aerosolzubereitung beschrieben die Insulin als Wirkstoff enthält.

### Vorbereitung der Insulinlösung und Befüllung des Verneblers.

175 mg kristallisiertes Insulin (Natriumsalz) vom Rind (enstprechend 4462.6 I.E. nach Angabe des Herstellers) wurden in 3.5 ml sterilem gereinigtem Wasser (Seralpur®-Wasser) gelöst. Anschließend wurde unter leichtem Rühren 8.5 µl m-Cresol (entsprechend 8,65 mg) und 7,53 mg Phenol, gelöst in 100 µl sterilem, greinigtem Wasser, hinzugefügt. Zu dieser Lösung wurden 365 µl einer 5 mg/ml ZnCl₂-Lösung gegeben (entsprechend einem Gewichtsanteil von 0.5% Zink bezogen auf die eingesetzte Insulinmenge) und der pH Wert auf 7.4 mit 0.2 N NaOH eingestellt. Das Volumen des Gemisches wurde auf 5 ml mit sterilem, gereinigtem Wasser aufgefüllt und durch einen sterilen Milliporefilter (Porengröße 0.22 µm) filtriert. 4,5 ml der Aerosolzubereitung wurden in den Vorratsbehälter (72 Figur 4) des Verneblers (Respimat) überführt. Der Behälter wurde mit einer Kappe verschlossen und das Device mit dem Behälter bestückt.

Die so hergestellte Aerosolzubereitung weist eine Konzentration von ca. 35mg/ml Insulin auf, wobei die Viskosität dieser Lösung ca. 1020·10⁻⁶ Pa · s beträgt.

### In vivo Versuch mit dem Respimat® und hoch-konzentrierter Insulinlösung

Die Applikation des Insulins beim Hund erfolgte unter Pentobarbitalnarkose nach vorheriger Basissedation. Das Tier wurde intubiert und künstlich beatmet wie vorher. Insgesamt wurden 6 Hübe Insulinlösung vom Respimat Gerät geliefert. Jeder Hub wurde zu Beginn einer Inspiration appliziert. Zwischen der Inspirationsphase und der Expiration war eine Pause von 5 Sekunden. Vor der nächsten Insulinapplikation wurden dem Tier zwei Atemzyklen ohne Beeinflüßung erlaubt. Blut wurde 1 Std. vor der Applikation, gleichzeitig mit der Applikation und zu verschiedenen Zeitpunkten danach über 8 Stunden entnommen. Der Blutglucosespiegel wurde im frischen Blut nach der Methode von Trasch, Koller und Tritschler (Klein. Chem. 30; 969 [1984]) mit Hilfe eines Refletron®-gerätes (Boehringer Mannheim) gemessen. Überraschenderweise wurde auch mit dieser hochkonzentrierten Insulinlösung eine gute biologischen Wirksamkeit (Senkung des

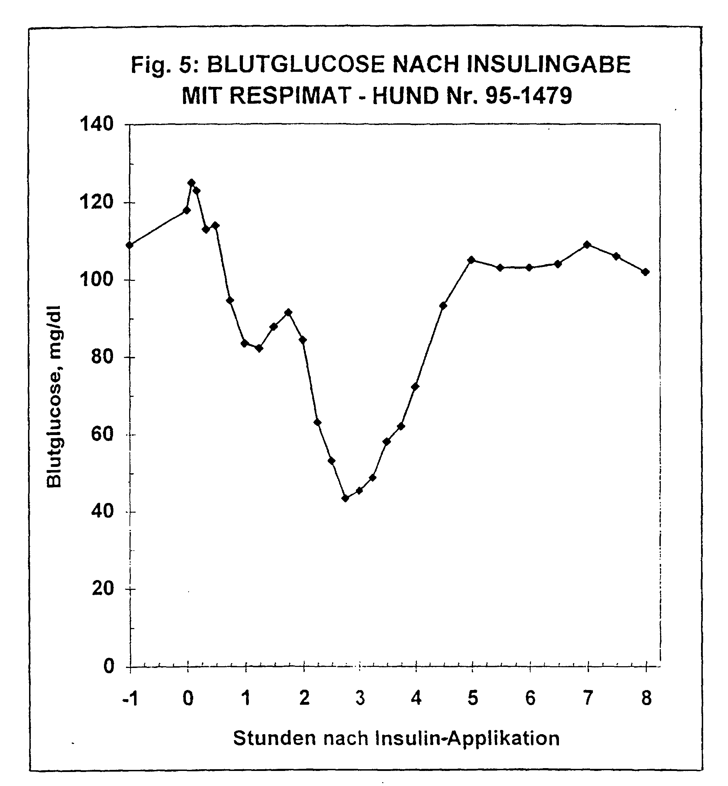

Blutglucosespiegels nach inhalative Insulingabe) gefunden. Fig. 5 illustriert dieses Ergebnis.

Die erfindungsgemäßen wäßrigen Aerosolzubereitungen können falls erforderlich neben dem Wirkstoff und Wasser weitere Lösemittel - wie z.B. Ethanol - enthalten. Die Menge an Ethanol wird in Abhängigkeit von den Löseeigenschaften der Wirkstoffe dadurch begrenzt, daß bei zu hohen Konzentrationen der Wirkstoff aus der Aerosolzubereitung ausfallen kann. Zusätze zur Stabilisierung der Lösung wie beispielsweise pharmakologisch unbedenkliche Konservierungsmittel wie z.B. Ethanol, Phenol, Kresol oder Paraben, pharmakologisch verträgliche Säuren, Basen oder Puffer zur Einstellung des pH-Wertes oder Surfactants sind möglich. Des weiteren ist zur Stabilisierung der Lösung oder zur Verbesserung der Aerosolqualität der Zusatz eines Metall-Chelatbildners - wie z. B. EDTA - möglich. Zur Verbesserung der Löslichkeit und / oder der Stabilität des Wirkstoffes in der Aerosolzubereitung können Aminosäuren, wie Aspartsäure, Glutamsäure (Asparagin, Glutamin) und insbesondere Prolin zugesetzt werden.

Wie erwähnt, verkörpern folgende Wirkstoffe der erfindungsgemäßen Arzneimittelzubereitung bevorzugte Wirkstoffe zusätzlich zu Interferonen, Superoxid Disumtase und Insulin:
Antisense Oligonukleotide
Orexine
Erythropoetin
Tumor Nekrose Faktor-alpha
Tumor Nekrose Faktor-beta
GM-CSF ("Granulocyte-macrophage colony stimulating factor")
Annexine
Calcitonin
Leptine
Parathormone
Parathormone Fragment
Interleukine wie z.B.,Interleukin 2, Interleukin10, Interleukin 12
Lösliches ICAM ("Intercellular adhesion molecule")
Somatostatin
Somatotropin
tPA ("Tissue plasminogen activator")
TNK-tPA
Tumorassozierte Antigene (als Peptid, Protein oder als DNA)
Peptid Bradykinin Antagonisten
Urodilatin
GHRH (Growth hormone releasing hormone)
CRF (Corticotropin releasing factor)
EMAP II
Heparin
Lösliche Interleukin Rezeptoren wie sIL-1 Rezeptor
Impfstoffe wie Hepatitis Vakzine oder Masern Vakzine
Antisense Polynukleotide
Transkriptions Faktoren.

## Patentansprüche

1. Treibgasfreier Vernebler enthaltend eine Aerosolzubereitung in Form einer Lösung mit Wasser oder einem Wasser-Ethanol-Gemisch als Lösungsmittel, welche einen Wirkstoff in einer Konzentration zwischen 25 und 100 mg/ml, der ausgewählt ist aus der Gruppe
Antisense Oligonukleotide
Orexine
Eryhtropoetin
Tumor Nekrose Faktor-alpha
Tumor Nekrose Faktor-beta
GM-CSF ("Granulocyte-macrophage colony stimulating factor")
Annexine
Calcitonin
Leptine
Parathormone
Parathormone Fragement
Interleukine wie z.B., Interleukin 2, Interleukin 10, Interleukin 12
Insulin
Superoxid Dismutase
ein Interferon
Lösliches ICAM ("Interceullar adhesion molecule")
Somatostatin
Somatotropin
tPA("Tissue plasminogen activator")
TNK-tPA
Tumorassozierte Antigene (als Peptid, Protein oder als DNA)
Peptid Bradykinin Antagonisten
Urodilatin
GHRH (Growth hormone releasing hormone)
CRF (Corticotropin releasing factor)
EMAP II
Heparin
Lösliche Interleukin Rezeptoren wie sIL-1 Rezeptor
Impfstoffe wie Hepatitis Vakzine oder Masern Vakzine
Antisense Polynukleotide
Transkriptions Faktoren,
enthält, wobei der treibgasfreie Vernebler geeignet ist, eine therapeutisch wirksame Menge einer Einzeldosierung der Aerosolzubereitung in einer Meßkammer abzumessen und unter hohem Druck zwischen 100 und 500 bar durch mindestens eine Düse mit einem hydraulischen Durchmesser von 1 bis 12 Mikrometer zu inhalierbaren Tröpfchen mit einer mittleren Teilchengröße von kleiner als 10 Mikrometer zu versprühen.

2. Treibgasfreier Vernebler nach Anspruch 1, **dadurch gekennzeichnet, daß** der Wirkstoff Insulin ist.

3. Treibgasfreier Vernebler nach Anspruch 2, **dadurch gekennzeichnet, daß** der Wirkstoff Insulin in einer Konzentration von 25 bis 60 mg/ml verwendet wird.

4. Treibgasfreier Vernebler nach Anspruch 2, **dadurch gekennzeichnet, daß** der Wirkstoff Insulin in einer Konzentration größer 30mg/ml verwendet wird.

5. Treibgasfreier Vernebler nach Anspruch 2, **dadurch gekennzeichnet, daß** der Wirkstoff Insulin in einer Konzentration von 30 bis 60 mg/ml verwendet wird.

6. Treibgasfreier Vernebler nach Anspruch 2, **dadurch gekennzeichnet, daß** der Wirkstoff Insulin in einer Konzentration von 33 bis 40 mg/ml verwendet wird.

7. Treibgasfreier Vernebler nach Anspruch 1, **dadurch gekennzeichnet, daß** der Wirkstoff Superoxid Dismutase ist.

8. Treibgasfreier Vernebler nach Anspruch 1, **dadurch gekennzeichnet, daß** der Wirkstoff ein Interferon ist.

9. Treibgasfreier Vernebler nach Anspruch 1, **dadurch gekennzeichnet, daß** der Wirkstoff ein Interferon Omega ist.

10. Treibgasfreier Vernebler nach einem der Ansprüche 1 bis 9, **dadurch gekennzeichnet, daß** die Aerosolzubereitung einen oder mehrere Hilfsstoffe aus der Gruppe der oberflächenaktiven Stofe, wie Surfactants, Emulgatoren, Stabilisatoren, Permeationsverbesserer und/oder Konservierungsstoffe enthält.

11. Treibgasfreier Vernebler nach Anspruch 1 bis 10, **dadurch gekennzeichnet, daß** die Aerosolzubereitung eine Aminosäure enthält.

12. Treibgasfreier Vernebler nach Anspruch 11, **dadurch gekennzeichnet, daß** die Aerosolzubereitung Prolin, Aspartsäure oder Glutamsäure geeignet zur Verbesserung der Löslichkeit oder der Stabilität des Wirkstoffes enthält.

13. Treibgasfreier Vernebler nach Anspruch 1, **dadurch gekennzeichnet, daß** die Aerosolzubereitung bei 25°C eine Viskosität von bis zu 1600·10⁻⁶ Pa·s, gemessen mit einem Oswald Viskosimeter, aufweist.

14. Treibgasfreier Vernebler nach Anspruch 1, **dadurch gekennzeichnet, daß** die Aerosolzubereitung bei 25°C eine Viskosität zwischen 900·10⁻⁶ und 1600 · 10⁻⁶ Pa·s, gemessen mit einem Oswald Viskosimeter, aufweist.

15. Treibgasfreier Vernebler nach Anspruch 1, **dadurch gekennzeichnet, daß** die Aerosolzubereitung bei 25°C eine Viskosität zwischen 900·10⁻⁶ und 1100 · 10⁻⁶ Pa·s, gemessen mit einem Oswald Viskosimeter, aufweist.

16. Treibgasfreier Vernebler nach Anspruch 1, **dadurch gekennzeichnet, daß** die Aerosolzubereitung bei 25°C eine Viskosität zwischen 950·10⁻⁶ und 1300 · 10⁻⁶ Pa·s, gemessen mit einem Oswald Viskosimeter, aufweist.

17. Treibgasfreier Vernebler nach Anspruch 13 oder 15, **dadurch gekennzeichnet, daß** der Wirkstof Superoxid Dismutase ist.

18. Treibgasfreier Vernebler nach Anspruch 13 oder 15, **dadurch gekennzeichnet, daß** der Wirkstoff ein Interferon ist, bevorzugt Interferon Omega.

19. Treibgasfreier Vernebler nach Anspruch 13 oder 15, **dadurch gekennzeichnet, daß** der Wirkstoff Insulin ist.

20. Treibgasfreier Vernebler nach einem der Ansprüche 1 bis 19, **dadurch gekennzeichnet, dass** der Vernebler klein genug ist, um in einer Jackentasche oder Handtasche ständig getragen zu werden.

21. Treibgasfreier Vernebler nach einem der Ansprüche 1 bis 19, **dadurch gekennzeichnet, dass** der Vernebler einen Düsenkörper mit zwei Düsenöffnungen aufweist, die so gerichtet sind, daß sich die beiden generierten Strahlen so treffen, daß die Aerosolzubereitung vernebelt wird.

22. Verfahren zur Herstellung von treibgasfreien Aerosolen für die inhalative Applikation, **dadurch gekennzeichnet, daß** in einem treibgasfreien Vernebler eine therapeutisch wirksame Menge einer Einzeldosierung einer Aerosolzubereitung nach einem der Ansprüche 1 bis 20 in einer Meßkammer abgemessen und unter hohem Druck zwischen 100 und 500 bar durch mindestens eine Düse mit einem hydraulischen Durchmesser von 1 bis 12 Mikrometer zu inhalierbaren Tröpfchen mit einer mittleren Teilchengröße kleiner als 10 Mikrometer versprüht wird.

23. Verfahren nach Anspruch 22, **dadurch gekennzeichnet, daß** die Aerosolzubereitung innerhalb einer Zeit zwischen einer und zwei Sekunden vernebelt wird.

24. Verfahren nach einem der Ansprüche 22 oder 23, **dadurch gekennzeichnet, daß** die wirksame Menge der Einzeldosierung zwischen 10 und 20 Mikroliter beträgt.

25. Verfahren nach einem der Ansprüche 22 bis 24, **dadurch gekennzeichnet, daß** der Vernebler einen Düsenkörper mit zwei Düsenöffnungen aufweist, die so gerichtet sind, daß sich die beiden generierten Strahlen so treffen, daß die Aerosolzubereitung vernebelt wird.

26. Verfahren nach einem der Ansprüche 22 bis 25, **dadurch gekennzeichnet, daß** der Wirkstoff Insulin ist.

27. Verfahren nach Anspruch 26, **dadurch gekennzeichnet, daß** die Konzentration des Insulins zwischen 25 mg/ml und 60 mg/ml, bevorzugt zwischen 30 und 40 mg/ml liegt.

28. Verwendung eines treibgasfreien Verneblers nach einem der Ansprüche 1 bis 21 enthaltend eine Aerosolzubereitung nach einem der Ansprüche 1 bis 21 zur Herstellung eines Aerosols mit einer mittlerer Teilchengröße von unter 10 Mikrometer.

29. Verwendung nach Anspruch 27, **dadurch gekennzeichnet, dass** als Aerosolzubereitung eine wässrige Lösung mit mehr als 25 mg/ml Insulin, bevorzugt mehr als 30 mg/ml Insulin, stärker bevorzugt 30 bis 60 mg/ml Insulin, besonders bevorzugt 33 bis 40 mg/ml Insulin eingesetzt wird.

## Claims

1. Propellant-free nebulizer containing an aerosol preparation in the form of a solution with water or a water/ethanol mixture as solvent, which contains an active substance in a concentration between 25 and 100 mg/ml, which is selected from among:
antisense oligonucleotides
orexins
erythropoietin
tumour necrosis factor-alpha
tumour necrosis factor-beta
GM-CSF (granulocyte-macrophage colony stimulating factor)
annexins
calcitonin
leptins
parathyrin
parathyrin fragment
interleukins such as interleukin 2, interleukin 10, interleukin 12
insulin
superoxide dismutase
an interferon
soluble ICAM (intercellular adhesion molecule)
somatostatin
somatotropin
tPA (tissue plasminogen activator)
TNK-tPA
tumour-associated antigens (as peptide, protein or as DNA)
peptide bradykinin antagonists
urodilatin
GHRH (growth hormone releasing hormone)
CRF (corticotropin releasing factor)
EMAP II
heparin
soluble interleukin receptors such as sIL-1 receptor
vaccines such as hepatitis vaccine or measles vaccine
antisense polynucleotides
transcription factors,
wherein the propellant-free nebulizer is suitable for measuring a therapeutically active amount of a single dose of the aerosol preparation in a measuring chamber and of spraying it under high pressure between 100 and 500 bar through at least one nozzle with a hydraulic diameter of 1 to 12 microns to form inhalable droplets of a medium particle size of less than 10 micrometer.

2. Propellant-free nebulizer according to claim 1, **characterised in that** the active substance is insulin.

3. Propellant-free nebulizer according to claim 2, **characterised in that** the active substance insulin is used in a concentration of 25 to 60 mg/ml.

4. Propellant-free nebulizer according to claim 2, **characterised in that** the active substance insulin is used in a concentration greater than 30 mg/ml.

5. Propellant-free nebulizer according to claim 2, **characterised in that** the active substance insulin is used in a concentration of 30 to 60 mg/ml.

6. Propellant-free nebulizer according to claim 2, **characterised in that** the active substance insulin is used in a concentration of 33 to 40 mg/ml.

7. Propellant-free nebulizer according to claim 1, **characterised in that** the active substance is superoxide dismutase.

8. Propellant-free nebulizer according to claim 1, **characterised in that** the active substance is an interferon.

9. Propellant-free nebulizer according to claim 1, **characterised in that** the active substance is an interferon omega.

10. Propellant-free nebulizer according to one of claims 1 to 9, **characterised in that** the aerosol preparation contains one or more adjuvants from the group comprising the surface-active substances, such as surfactants, emulsifiers, stabilisers, permeation enhancers and/or preservatives.

11. Propellant-free nebulizer according to claims 1 to 10, **characterised in that** the aerosol preparation contains an amino acid.

12. Propellant-free nebulizer according to claim 11, **characterised in that** the aerosol preparation contains proline, aspartic acid or glutamic acid which is suitable for improving the solubility or stability of the active substance.

13. Propellant-free nebulizer according to claim 1, **characterised in that** the aerosol preparation has a viscosity of up to 1600. 10⁻⁶ Pa s at 25°C, measured with an Oswald viscosimeter.

14. Propellant-free nebulizer according to claim 1, **characterised in that** the aerosol preparation has a viscosity of between 900. 16⁻⁶ and 1600. 10⁻⁶ Pa s at 25°C, measured with an Oswald viscosimeter.

15. Propellant-free nebulizer according to claim 1, **characterised in that** the aerosol preparation has a viscosity of between 900. 16⁻⁶ and 1100. 10⁻⁶ Pa s at 25°C, measured with an Oswald viscosimeter.

16. Propellant-free nebulizer according to claim 1, **characterised in that** the aerosol preparation has a viscosity of between 950. 10⁻⁶ and 1300. 10⁻⁶ Pa s at 25°C, measured with an Oswald viscosimeter.

17. Propellant-free nebulizer according to claim 13 or 15, **characterised in that** the active substance is superoxide dismutase.

18. Propellant-free nebulizer according to claim 13 or 15, **characterised in that** the active substance is an interferon, preferably interferon omega.

19. Propellant-free nebulizer according to claim 13 or 15, **characterised in that** the active substance is insulin.

20. Propellant-free nebulizer according to one of claims 1 to 19, **characterised in that** the nebulizer is small enough to be carried constantly in a jacket pocket or handbag.

21. Propellant-free nebulizer according to one of claims 1 to 19, **characterised in that** the nebulizer has a nozzle body with two nozzles which are directed so that the two jets meet in such a way that the aerosol preparation is nebulized.

22. Process for preparing propellant-free aerosols for administration by inhalation, **characterised in that** in a propellant-free nebulizer a therapeutically active quantity of a single dose of an aerosol preparation according to one of claims 1 to 20 is measured in a measuring chamber and is sprayed under high pressure of between 100 and 500 bar through at least one nozzle with a hydraulic diameter of 1 to 12 µm to form inhalable droplets with an average particle size of less than 10 µm.

23. Process according to claim 22, **characterised in that** the aerosol preparation is nebulized within a period of between 1 and 2 seconds.

24. Process according to one of claims 22 or 23, **characterised in that** the effective quantity of the single dose is between 10 and 20 microlitres.

25. Process according to one of claims 22 to 24, **characterised in that** the nebulizer has a nozzle body with two nozzles directed so that the two jets meet in such a way that the aerosol preparation is nebulized.

26. Process according to one of claims 22 to 25, **characterised in that** the active substance is insulin.

27. Process according to claim 26, **characterised in that** the concentration of the insulin is between 25 mg/ml and 60 mg/ml, preferably between 30 and 40 mg/ml.

28. Use of a propellant-free nebulizer according to one of claims 1 to 21 containing an aerosol preparation according to one of claims 1 to 21 for preparing an aerosol with an average particle size of less than 10 µm.

29. Use according to claim 27, **characterised in that** the aerosol preparation used is an aqueous solution containing more than 25 mg/ml of insulin, preferably more than 30 mg/ml of insulin, more preferably 30 to 60 mg/ml of insulin, particularly preferably 33 to 40 mg/ml of insulin.

## Revendications

1. Nébuliseur sans gaz propulseur contenant une préparation pour aérosol sous forme d'une solution avec de l'eau ou un mélange eau-éthanol comme solvant, qui contient un principe actif en une concentration de 25 à 100 mg/ml, qui est choisi dans le groupe
oligonucléotides antisens,
oréxines
érythropoïétine,
facteur alpha de nécrose des tumeurs,
facteur bêta de nécrose des tumeurs,
GM-CSF (« granulocyte-macrophage colony stimulating factor »),
annexines,
calcitonine,
leptines,
parathormone,
fragment de parathormone,
interleukines comme par exemple interleukine 2, interleukine 10, interleukine 12,
insuline,
superoxyde dismutase,
un interféron,
ICAM (« intercellular adhesion molecule ») soluble,
somatostatine,
somatotropine,
tPA (« tissue plasminogen activator »),
TNK-tPA,
Antigènes associés à des tumeurs (sous forme de peptide, de protéine ou de DNA), antagonistes de bradykinine peptidiques,
urodilatine,
GHRH (growth hormone releasing hormone)
CRF (corticotropin releasing factor),
EMAP II
héparine,
récepteurs d'interleukines solubles comme le récepteur de sIL-1
vaccins comme le vaccin contre l'hépatite ou le vaccin contre la rougeole,
polynucléotides antisens,
facteurs de transcription,
où le nébuliseur sans gaz propulseur convient pour doser une quantité thérapeutiquement efficace d'une dose unique de la préparation pour aérosol dans une chambre de mesure et pour la pulvériser sous une haute pression entre 100 et 500 bar par au moins une buse ayant un diamètre hydraulique de 1 à 12 micromètres en gouttelettes inhalables ayant une grandeur moyenne des particules de moins de 10 micromètres.

2. Nébuliseur sans gaz propulseur selon la revendication 1 **caractérisé en ce que** le principe actif est l'insuline.

3. Nébuliseur sans gaz propulseur selon la revendication 2 **caractérisé en ce que** le principe actif insuline est utilisé en une concentration de 25 à 60 mg/ml.

4. Nébuliseur sans gaz propulseur selon la revendication 2 **caractérisé en ce que** le principe actif insuline est utilisé en une concentration supérieure à 30 mg/ml.

5. Nébuliseur sans gaz propulseur selon la revendication 2 **caractérisé en ce que** le principe actif insuline est utilisé en une concentration de 30 à 60 mg/ml.

6. Nébuliseur sans gaz propulseur selon la revendication 2 **caractérisé en ce que** le principe actif insuline est utilisé en une concentration de 33 à 40 mg/ml.

7. Nébuliseur sans gaz propulseur selon la revendication 1 **caractérisé en ce que** le principe actif est la superoxyde dismutase.

8. Nébuliseur sans gaz propulseur selon la revendication 1 **caractérisé en ce que** le principe actif est un interféron.

9. Nébuliseur sans gaz propulseur selon la revendication 1 **caractérisé en ce que** le principe actif est un interféron oméga.

10. Nébuliseur sans gaz propulseur selon l'une des revendications 1 à 9 **caractérisé en ce que** la préparation pour aérosol contient un ou plusieurs adjuvants du groupe des substances tensioactives, comme des surfactants, des émulsifiants, des stabilisants, des agents améliorant la perméation et/ou des conservateurs.

11. Nébuliseur sans gaz propulseur selon les revendications 1 à 10 **caractérisé en ce que** la préparation pour aérosol contient un aminoacide.

12. Nébuliseur sans gaz propulseur selon la revendication 11 **caractérisé en ce que** la préparation pour aérosol contient de la proline, de l'acide aspartique ou de l'acide glutamique convenant pour améliorer la solubilité ou la stabilité du principe actif.

13. Nébuliseur sans gaz propulseur selon la revendication 1 **caractérisé en ce que** la préparation pour aérosol présente à 25°C une viscosité pouvant atteindre 1600.10⁻⁶ Pa.s, mesurée avec un viscosimètre d'Oswald.

14. Nébuliseur sans gaz propulseur selon la revendication 1 **caractérisé en ce que** la préparation pour aérosol présente à 25°C une viscosité entre 900. 16⁻⁶ et 1600.10⁻⁶ Pa.s, mesurée avec un viscosimètre d'Oswald.

15. Nébuliseur sans gaz propulseur selon la revendication 1 **caractérisé en ce que** la préparation pour aérosol présente à 25°C une viscosité entre 900. 10⁻⁶ et 1100.10⁻⁶ Pa.s, mesurée avec un viscosimètre d'Oswald.

16. Nébuliseur sans gaz propulseur selon la revendication 1 **caractérisé en ce que** la préparation pour aérosol présente à 25°C une viscosité entre 950. 16⁻⁶ et 1300.10⁻⁶ Pa.s, mesurée avec un viscosimètre d'Oswald.

17. Nébuliseur sans gaz propulseur selon la revendication 13 ou 15 **caractérisé en ce que** le principe actif est la superoxyde dismutase.

18. Nébuliseur sans gaz propulseur selon la revendication 13 ou 15 **caractérisé en ce que** le principe actif est un interféron, de préférence un interféron oméga.

19. Nébuliseur sans gaz propulseur selon la revendication 13 ou 15 **caractérisé en ce que** le principe actif est l'insuline.

20. Nébuliseur sans gaz propulseur selon l'une des revendications 1 à 19 **caractérisé en ce que** le nébuliseur est suffisamment petit pour être porté constamment dans une poche de veste ou un sac à main.

21. Nébuliseur sans gaz propulseur selon l'une des revendications 1 à 19 **caractérisé en ce que** le nébuliseur comprend un corps de buses avec deux buses qui sont dirigées de telle manière que les deux jets se rencontrent de telle sorte que la préparation pour aérosol est nébulisée.

22. Procédé de production d'aérosols sans gaz propulseur pour l'application par inhalation **caractérisé en ce que**, dans un nébuliseur sans gaz propulseur, une quantité thérapeutiquement efficace d'une dose unique d'une préparation pour aérosol selon l'une des revendications 1 à 20 est dosée dans une chambre de mesure et est pulvérisée sous une haute pression entre 100 et 500 bar par au moins une buse ayant un diamètre hydraulique de 1 à 12 micromètres en gouttelettes inhalables ayant une taille de particule moyenne inférieure à 10 micromètres.

23. Procédé selon la revendication 22 **caractérisé en ce que** la préparation pour aérosol est nébulisée en une durée entre une et deux secondes.

24. Procédé selon l'une des revendications 22 ou 23 **caractérisé en ce que** la quantité efficace de la dose unique est entre 10 et 20 microlitres.

25. Procédé selon l'une des revendications 22 à 24 **caractérisé en ce que** le nébuliseur comprend un corps de buses avec deux buses qui sont dirigées de telle manière que les deux jets se rencontrent de telle sorte que la préparation pour aérosol est nébulisée.

26. Procédé selon l'une des revendications 22 à 25 **caractérisé en ce que** le principe actif est l'insuline.

27. Procédé selon la revendication 26 **caractérisé en ce que** la concentration de l'insuline est située entre 25 mg/ml et 60 mg/ml, de préférence entre 30 et 40 mg/ml.

28. Utilisation d'un nébuliseur sans gaz propulseur selon l'une des revendications 1 à 21 contenant une préparation pour aérosol selon l'une des revendications 15 à 21 pour la production d'un aérosol ayant une taille de particule moyenne inférieure à 10 micromètres.

29. Utilisation selon la revendication 27 **caractérisée en ce qu'**une solution aqueuse ayant plus de 25 mg/ml d'insuline, de préférence plus de 30 mg/ml d'insuline, de préférence encore 30 à 60 mg/ml d'insuline, de manière particulièrement préférée 33 à 40 mg/ml d'insuline, est utilisée comme préparation pour aérosol.
